# EUROPEAN PATENT APPLICATION

(11) **EP 4 445 854 A1**
(43) Date of publication of application: **16.10.2024**
(21) Application number: 22904439.1
(22) Date of filing: 14.10.2022
(51) Int. Cl.: A61B 18/14, A61B 90/00, A61B 18/00

(54) **CATHETER, CATHETER OPERATION METHOD, AND CATHETER SYSTEM**

(30) Priority: 09.12.2021 KR 20210175540
(71) Applicant: Park, Eul Joon, Seoul 06356 (KR)
(72) Inventor: Park, Eul Joon, Seoul 06356 (KR)
(74) Representative: Schiweck Weinzierl Koch Patentanwälte Partnerschaft mbB
(86) International application number: PCT/KR2022/015607
(87) International publication number: WO 2023/106605

(57) **Abstract**

Disclosed in an embodiment is a catheter, comprising: a first tube; and an electrode support portion disposed inside the first tube, wherein an opening area of the electrode support portion changes according to the extent to which a predetermined area of the electrode support portion is exposed by pulling the first tube proximally.

## Description

### [Technical Field]

Embodiments relate to a catheter, catheter operation method, and catheter system.

### [Background Art]

Denervation is a surgical procedure for blocking a part of nerve paths for various nerves such as sensory nerves and automatic nerves so that stimulation or information is not delivered. The denervation is increasingly being used for treatment of various diseases such as arrhythmia, pain relief, and plastic surgery.

In particular, as it has been recently reported that the denervation is effective in treating hypertension, endeavors are being made to apply the denervation as a method for effectively treating hypertension.

In case of hypertension, it is possible to control blood pressure with drugs in most cases, so to date, many hypertensive patients are treated for hypertension by relying on drugs. However, this method of lowering blood pressure using drugs has several problems, including the inconvenience of having to continuously take the drug, cost, and side effects such as organ damage due to long-term use of the drug. Moreover, some hypertensive patients suffer from intractable hypertension in which blood pressure cannot be controlled with drugs. Since such intractable hypertension cannot be treated with drugs, there is a high risk of causing accidents such as stroke, arrhythmia, kidney disease, etc. to patients, so treatment of intractable hypertension is a very serious and urgent problem.

In such situations, the denervation attracts attention as an innovative scheme to treat hypertension. In particular, the denervation for treating hypertension may be performed by ablating nerves around renal nerves to inactivate nerve conduction so that the nerves are blocked.

For example, a representative method of blocking renal to treat hypertension is to use a catheter. The denervation using a catheter can be performed by inserting a catheter into a part of a human body, for example the thigh, and then, generating heat through radio frequency (RF) energy, etc. at the distal end of the catheter in a state in which the distal end of the catheter is placed in the renal artery along the blood vessel, so that the sympathetic nerves around the renal artery are blocked.

Furthermore, these catheters can be used for denervation in various parts of the body (liver, brain, heart, etc.), as well as in the kidneys for the treatment of hypertension.

For example, when treating diabetes, nerve ablation must be performed on the hepatic artery. In this case, the hepatic artery has many curved blood vessels compared to the renal artery, so in order to perform ablation for denervation in various parts of the body (e.g., hepatic artery) described above, improved flexibility and reduced diameter are required compared to existing catheters.

In addition, since this type of denervation using a catheter makes a much smaller incision than denervation through abdominal operation, there are advantages that latent complications and side effects are significantly reduced, and the treatment and recovery time due to local anesthesia is very short. Therefore, it is spotlighted as a next-generation hypertension treatment method.

However, catheter-related techniques for the application to denervation are not yet sufficiently developed and thus there is much room for improvement.

In particular, the catheter should have a very small size since it may freely move in a blood vessel. However, in a conventional technique, it is very difficult to design the catheter with a small size.

Moreover, the head portion of conventionally developed or proposed catheters is equipped with one or more electrodes and various devices for sensing, and also includes various wires for transmitting power or electrical signals to these electrodes and sensing devices. Therefore, it is very difficult in the conventional technique to manufacture a catheter with a small size, which includes all of the above components.

In addition, in the case of some catheters, when the distal end of the catheter reaches treatment sites, the head portion is configured to expand so that the electrode approaches the inner wall of the blood vessel. In this case, in order for an operator to perform the expansion/contraction operation of the catheter head, a separate operation member may be provided long from the distal end to proximal end of the catheter. In this case, the internal structure of the catheter becomes more complicated and larger in size due to these operating members, which may add to the difficulty in miniaturizing the catheter. In addition, the operating member for operation is often made of a metal material, and various problems may occur when the operating member for operation is exposed to the outside at the head portion and comes into contact with blood.

### [Disclosure]

### [Technical Problem]

Embodiments according to the present invention have been created to solve the above-mentioned problems, and the object is to provide a catheter capable of being easily used even in blood vessels with small diameters or tortuous blood vessels.

In addition, it is possible to provide a catheter that has a simplified structure and is easy to miniaturize.

In addition, it is possible to provide a catheter that is easy to operate and easy to manufacture because there is no separate member in the catheter head to increase the diameter or area of an electrode.

The problems to be solved in the embodiment is not limited to the above, and also includes objects and effects that can be understood from the means for solving the problem or the embodiment described below.

### [Technical Solution]

A catheter according to an embodiment comprises a first tube; and an electrode support portion disposed inside the first tube, wherein an opening area of the electrode support portion changes according to an extent to which a predetermined area of the electrode support portion is exposed by pulling the first tube proximally.

The opening area of the electrode support portion may change after the electrode portion is exposed.

The electrode support portion may include a distally disposed first area; a second area S2 in contact with a proximal end of the first area and extending proximally and inside of the first tube; and a third area in contact with a proximal end of the second area and extending proximally.

The electrode support portion may include a plurality of electrode support portions, wherein the plurality of electrode support portions includes a first electrode support portion, a second electrode support portion, and a third electrode support portion spaced apart from each other.

The first area of the first electrode support portion may have a length equal to or different from at least one of the first area of the second electrode support portion and the first area of the third electrode support portion in a longitudinal direction.

The electrode portion may include a plurality of electrode portions, wherein the plurality of electrodes may include a first electrode disposed in the first area of the first electrode support portion; a second electrode disposed in the first area of the second electrode support portion; and a third electrode disposed in the first area of the third electrode support portion.

The first electrode, the second electrode, and the third electrode may be disposed to be spaced apart in a longitudinal direction.

The first electrode, the second electrode, and the third electrode may be disposed to be offset from each other in a plane intersecting a longitudinal direction.

The first electrode support portion, the second electrode support portion, and the third electrode support portion may be disposed to have the same angle with respect to a center of the first tube.

The catheter may comprise a second tube disposed inside the first tube.

The electrode support portion may be disposed between the first tube and the second tube.

The catheter may further comprise a marker disposed in the second area.

The marker may be disposed along predetermined intervals.

The opening area may change in the first area or the second area.

The second area may have at least one of a stepped structure and an inclined structure.

The opening area may change in response to the extent of exposure of the first area or second area.

The opening area may increase as the extent of exposure of the first area or second area increases.

The opening area may not change after exposure of the third area.

A catheter system according to an embodiment comprises a catheter; and a handle connected to the proximal end of the catheter, wherein the catheter comprises a first tube; and an electrode support portion disposed inside the first tube, wherein the electrode support portion changes an opening area by pulling the first tube proximally.

The opening area may be changed by pulling the handle proximally.

A method for operating a catheter according to an embodiment comprises the steps of inserting a first tube; and controlling the opening area of the electrode support portion disposed within the first tube by pulling the first tube proximally.

### [Advantageous Effects]

The embodiments according to the present invention have been created to solve the above-mentioned problems, and can implement a catheter that has a simplified structure and is easy to miniaturize.

In addition, it is possible to implement a catheter that can be easily used even in blood vessels with small diameters or tortuous blood vessels.

In addition, it is possible to implement a catheter that is easy to operate and easy to manufacture because there is no separate member in the catheter head to increase the diameter or area of an electrode.

The various and beneficial advantages and effects of the present invention are not limited to the above-described content, and may be more easily understood through description of specific embodiments of the present invention.

### [Description of Drawings]

FIG. 1 is a conceptual diagram of a catheter system according to an embodiment,
FIG. 2 is a diagram of a catheter according to a first embodiment,
FIG. 3 is an enlarged view of part K in FIG. 2,
FIG. 4 is a diagram according to a first state of a catheter according to a first embodiment,
FIG. 5 is a view cut along AA' in FIG. 4,
FIG. 6 is a diagram of the catheter viewed from direction A1 in FIG. 4,
FIG. 7 is a diagram according to a second state of a catheter according to a first embodiment,
FIG. 8 is a diagram of the catheter viewed from direction A2 in FIG. 7,
FIG. 9 is a view cut along BB' in FIG. 7,
FIG. 10 is a view cut along CC' in FIG. 7,
FIG. 11 is a view cut along DD' in FIG. 7,
FIG. 12 is a diagram according to a third state of a catheter according to a first embodiment,
FIG. 13 is a diagram of the catheter viewed from direction A3 in FIG. 12,
FIG. 14 is a diagram of a catheter according to a modified example,
FIG. 15 is a diagram of the catheter viewed from direction A4 in FIG. 14,
FIG. 16 is a diagram of a catheter according to a second embodiment,
FIG. 17 is a diagram of a catheter according to another modified example,
FIG. 18 is an illustrative example of the catheter inserted into the renal artery according to an embodiment,
FIG. 19 is an illustrative diagram of the catheter inserted into the hepatic artery according to an embodiment,
FIG. 20 is a diagram of a catheter according to a third embodiment;
FIG. 21 is a diagram of a catheter according to a fourth embodiment;
FIG. 22 is a diagram of a catheter according to a fifth embodiment;
FIG. 23 is a diagram for explaining operations of a catheter and handle according to an embodiment,
FIG. 24 is a flowchart of a method for operating a catheter according to an embodiment.

### [Mode for Invention]

Since various modifications may be made to the present disclosure and the present disclosure may have various embodiments, particular embodiments will be illustrated in the drawings and described. However, this does not limit the present disclosure to the particular embodiments, and all modifications, equivalents, and substitutes included in the spirit and scope of the present disclosure should be construed as belonging to the present disclosure.

Terms including ordinal numbers such as first and second may be used to describe various elements, but the elements are not limited by the terms. The terms are only used for the purpose of distinguishing one element from another element. For example, a second element may be referred to as a first element while not departing from the scope of the present disclosure, and likewise, a first element may also be referred to as a second element. The term and/or includes a combination of a plurality of related described items or any one item among the plurality of related described items.

When it is mentioned that a certain element is "connected" or "coupled" to another element, although the certain element may be directly connected or coupled to the other element, it should be understood that another element may exist therebetween. On the other hand, when it is mentioned that a certain element is "directly connected" or "directly coupled" to another element, it should be understood that other elements do not exist therebetween.

Terms used in the application are merely used to describe particular embodiments and are not intended to limit the present disclosure. A singular expression includes a plural expression unless the context clearly indicates otherwise. In the application, terms such as "include" or "have" should be understood as designating that features, number, steps, operations, elements, parts, or combinations thereof exist and not as precluding the existence of or the possibility of adding one or more other features, numbers, steps, operations, elements, parts, or combinations thereof.

Unless otherwise defined, all terms including technical or scientific terms used herein have the same meaning as commonly understood by one of ordinary skill in the art to which the disclosure pertains. Terms, such as those defined in commonly used dictionaries, should be construed as having a meaning that is consistent with their meaning in the context of the relevant art and are not to be construed in an idealized or overly formal sense unless expressly so defined herein.

Hereinafter, an embodiment will be described in detail with reference to the accompanying drawings while like reference numerals will be given to the same or corresponding elements regardless of signs in the drawings and overlapping descriptions thereof will be omitted.

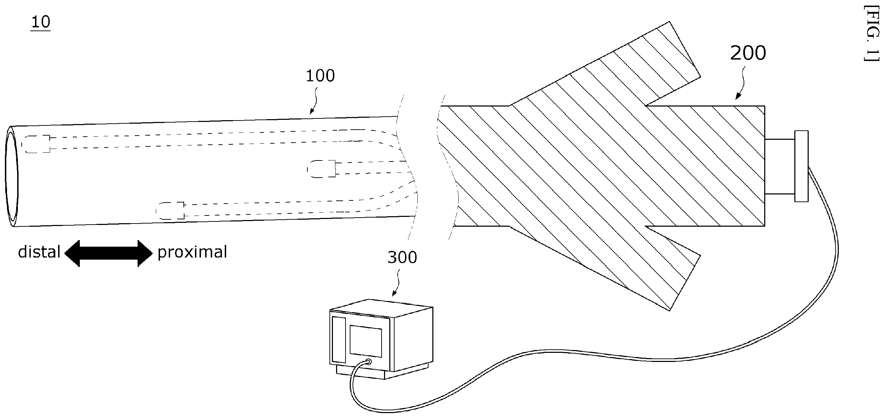

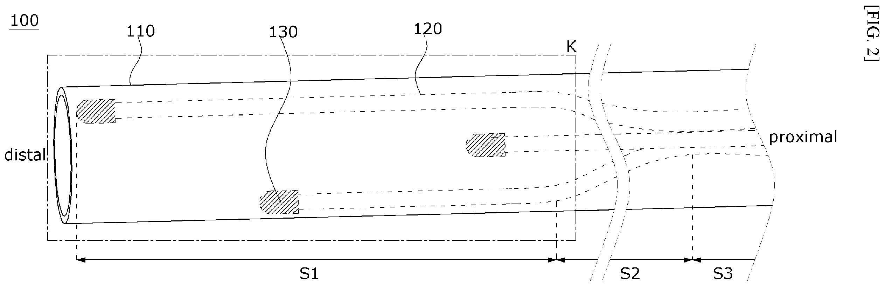

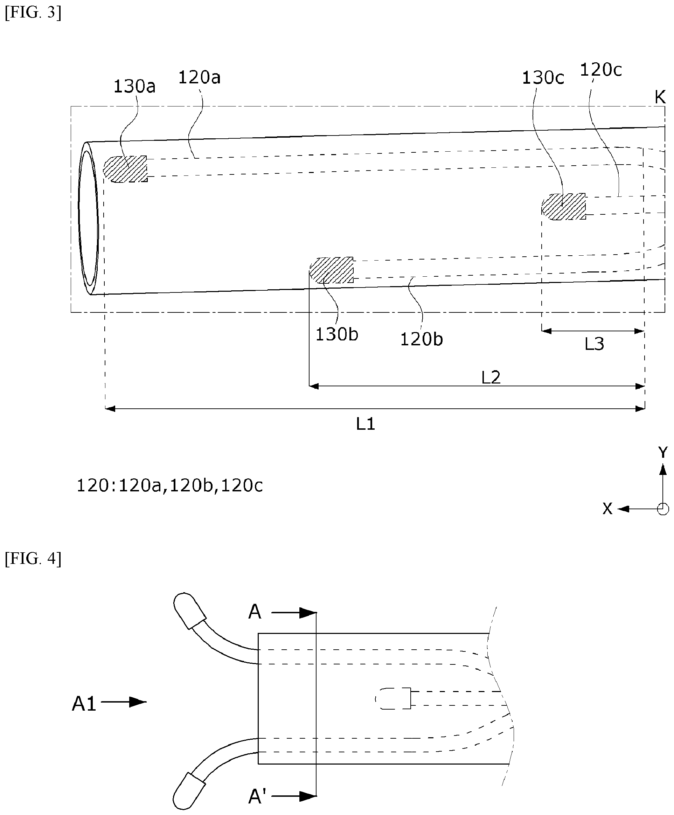

FIG. 1 is a conceptual diagram of a catheter system according to an embodiment, FIG. 2 is a diagram of a catheter according to a first embodiment, and FIG. 3 is an enlarged view of part K in FIG. 2.

Referring to FIG. 1, a catheter system 10 according to an embodiment may include a catheter 100, a handle 200, and a console 300.

The catheter 100 may be connected to the handle 200 and may include a plurality of electrodes in the distal area. This catheter 100 may be moved to a blood vessel (e.g., artery) connected to an organ (e.g., kidney or liver, etc.).

Furthermore, energy may be transferred to the electrodes of the catheter 100 according to manipulation by a user or operator through the handle 200. For example, energy that generates high temperature may be transferred to the electrode through the user's manipulation. Accordingly, the electrode of the catheter 100 may transfer energy to the nerves around the blood vessels. In addition, denervation around blood vessels may be achieved by the energy transferred.

The console 300 may be connected to the handle 200 and catheter 100. The console 300 may provide energy to the electrodes of the catheter 100. Furthermore, the handle 200 may adjust the size and form of energy provided to the electrodes of the catheter 100 in various ways by the user's manipulation or manipulation of the console 300. For example, the console 300 may include or be connected to an energy generator that generates energy (e.g., ultrasound, heat). Furthermore, the energy generator may provide various types of energy, such as pulse energy, ultrasonic energy, microwave energy, optical energy, heat energy, and radiation.

In addition, the handle 200 may be located between the console 300 and the catheter 100. The handle 200 may be located at the proximal end of catheter 100.

In addition, like the console 300, the handle 200 may be located outside the patient and may include a controller for ease of operation by the user. For example, the controller may be implemented in various ways, such as buttons and jogs.

In addition, the user may control the operation of the catheter 100, etc. through the handle 200. That is, when a command corresponding to the user's operation is generated, the generated command is transmitted to the console 300 or catheter 100, and the operation corresponding to the command may be performed in the console 300 or catheter 100.

Then, energy is transferred to the electrodes of the catheter 100 by the user's manipulation (for example, turning on a button for energy injection), and denervation may be performed with the transferred energy. For example, the electrode of the catheter 100 may be expanded to the outside of a first tube or into the blood vessel by the user or operator manipulating or driving the handle 200, or moving or pulling the first tube to the proximal end as described later. In addition, the electrodes may be inserted into the first tube by pulling the electrode support portion to the proximal end while fixing the first tube. Alternatively, the electrodes may be inserted into the first tube by pushing the first tube to the distal end while fixing the electrode support portion.

Further, in this specification, the distal end of the catheter refers to a side that reaches a treatment site among both ends in the longitudinal direction (X-axis direction) of the catheter. The distal end of the catheter is used interchangeably with `distal end portion', `distal part', and `distal end'. On the contrary, the proximal end of the catheter refers to the user's side among both ends of the catheter in the longitudinal direction (X-axis direction). In this case, the proximal end of the catheter located on the operator's side is used interchangeably with 'proximal end portion', 'proximal part', and 'proximal end'. In addition, the catheter has both ends extending long in one direction, and may be configured to move along the internal space of a blood vessel or the like.

Referring to FIGS. 2 and 3, the catheter 100 according to an embodiment may include a first tube 110, an electrode support portion 120, and an electrode portion 130.

First, the first tube 110 may include a hollow body. The first tube 110 may be made of various materials that are harmless to the human body. Furthermore, the first tube 110 may be made of a flexible material.

The electrode support portion 120 may be disposed inside the first tube 110. That is, the electrode support portion 120 may be located within the hollow of the first tube. For example, the electrode support portion 120 may be made of an elastic material. In addition, the electrode support portion 120 may be made of shape memory alloy (SMA), but is not limited to these materials.

In an embodiment, the electrode support portion 120 may change its opening area by pulling the first tube 110 proximally. As will be described later, the opening area may change by exposing a specific area (first area or second area) of the electrode support portion 120. Further, when another area (third area) of the electrode support portion 120 is exposed, the opening area may be substantially maintained.

The electrode portion 130 may be disposed on the electrode support portion 120. For example, the electrode portion 130 may be located in the first area S1 of the electrode support portion 120. Further, the electrode portion 130 may be located in a form surrounding the outer surface of the electrode support portion 120 or the circumference of the electrode support portion 120. Alternatively, the electrode portion 130 may be located at the end of the electrode support portion 120. Hereinafter, a structure in which the electrode portion 130 is located at the end of the electrode support portion 120 and surrounds the electrode support portion 120 will be described. In addition, the electrode portion 130 may be electrically connected to a wire (not shown) inside or outside the electrode support portion 120. In addition, wires (not shown) may be electrically connected to the handle or above-described console.

In detail, the electrode support portion 120 may include at least one electrode support portion. In this specification, the electrode support portion 120 will be described as including a first electrode support portion 120a, a second electrode support portion 120b, and a third electrode support portion 130c.

Further, the electrode support portion 120 may include a distally disposed first area S1, a second area S2 in contact with the proximal end of the first area S 1, and a third area S3 in contact with the proximal end of the second area S2. That is, the third area S3, second area S2, and first area S1 may be located sequentially along the first direction (X-axis direction). In this specification, the first direction (X-axis direction) may be a direction parallel to the longitudinal direction. In addition, the second direction (Y-axis direction) may be perpendicular to the first direction (X-axis direction), and the third direction (Z-axis direction) may be perpendicular to the first and second directions.

The second area S2 may extend inside and proximally of the first tube 110. Accordingly, the second area S2 may have at least one of a stepped structure and an inclined structure. In addition, the first area S1 may be located outside a portion of the second area S2. In addition, the first area S1 may be disposed outside the third area S3. Conversely, the third area S3 may be located inside the first tube 110 than the first area S 1. In addition, the third area S3 may be located inside compared to a partial area of the second area S2.

In addition, the plurality of electrode support portions 120a to 120c may have different or the same length in the longitudinal direction (X-axis direction) in the first area S1. For example, as shown, the plurality of electrode support portions 120a to 120c may have different lengths in the longitudinal direction (X-axis direction) in the first area S1.

For example, a length L1 in the first direction or longitudinal direction (X-axis direction) of the first electrode support portion 120a may be longer than a length L2 of the second electrode support portion 120b or length L3 of the third electrode support portion 120c. In addition, the length L2 in the first direction or longitudinal direction (X-axis direction) of the second electrode support portion 120b may be longer than the length L1 of the first electrode support portion 120a or length L3 of the third electrode support portion 120c.

The electrode portion 130 may include at least one electrode. The electrode may be located in the first area S1 of the electrode support portion 120. Accordingly, as will be described later, in case that the opening of the electrode support portion 120 or first area S1 is larger than the hollow opening of the first tube 110, energy may be transferred to the nerve adjacent to the blood vessel.

In an embodiment, the electrode portion 130 may include a first electrode 130a, a second electrode 130b, and a third electrode 130c. The first electrode 130a may be connected to the first electrode support portion 120a. In particular, the first electrode 130a may be located in the first area of the first electrode support portion 120a. The second electrode 130b may be connected to the second electrode support portion 120b. In particular, the second electrode 130b may be located in the first area of the second electrode support portion 120b. The third electrode 130c may be connected to the third electrode support portion 120c. In particular, the third electrode 130c may be located in the first area of the third electrode support portion 120c.

The first electrode 130a, second electrode 130b, and third electrode 130c may receive energy as described above. In addition, the first electrode 130a, second electrode 130b, and third electrode 130c may be disposed to be spaced apart from each other in the first direction (X-axis direction) or longitudinal direction. That is, the first electrode 130a, second electrode 130b, and third electrode 130c may be disposed to be offset from each other in a plane YZ intersecting the first direction (X-axis direction). Alternatively, the first electrode 130a, second electrode 130b, and third electrode 130c may not overlap each other in the plane YZ intersecting the first direction (X-axis direction). With this configuration, the electrode support portion 120 where the electrode portion 130 is located may be easily located inside the first tube 110. On the other hand, in case that a plurality of electrodes overlap on the plane YZ, the radius or diameter of the first tube 110 inevitably increases. Accordingly, the catheter 100 according to an embodiment may provide the first tube 110 with a plurality of electrodes and a small radius or diameter. In addition, the catheter 100 may be easily inserted into blood vessels with a small radius or diameter. Accordingly, procedures such as resection within blood vessels may be performed more easily.

In addition, the opening area of the electrode support portion 120 may be changed by pulling the first tube 110 proximally. In this case, the opening area may change as a specific area (e.g., first area or second area S2) of the electrode support portion 120 is exposed. For example, the distal end of the first tube 110 may change as the first tube 110 moves in an area where the distal end of the first tube 110 is in contact with the first area S1 or second area S2. In this specification, the opening area may correspond to the distance or radius from the center of the first tube 110, which will be described later. For example, the opening area of the first tube 110 may correspond to the distance from the center to inner surface of the first tube 110, the radius, diameter, or hollow area. In addition, the opening area of the electrode support portion 120 may be the distance from the center of the first tube 110 to the first area S1 of the electrode support portion 120, radius, or area (e.g., the area of a circle with the above distance as the radius). For example, when the first tube 110 is pulled proximally, the radius from the center of the first tube 110 to the first area S1 of the electrode support portion 120 may change, and the radius of the first tube 110 may be fixed.

Accordingly, the catheter 100 according to an embodiment may not have a separate operating member, for example in the form of a driving tube or wire, for expanding the electrode support portion 120 outward. Nonetheless, the catheter 100 may extend the electrodes that perform the ablation outward. Furthermore, since there is no separate operating member as described above, the catheter 100 according to an embodiment may be easily used even in small-diameter blood vessels by reducing the diameter of the first tube 110. In other words, the catheter 100 according to an embodiment may expand the range of organs to be treated.

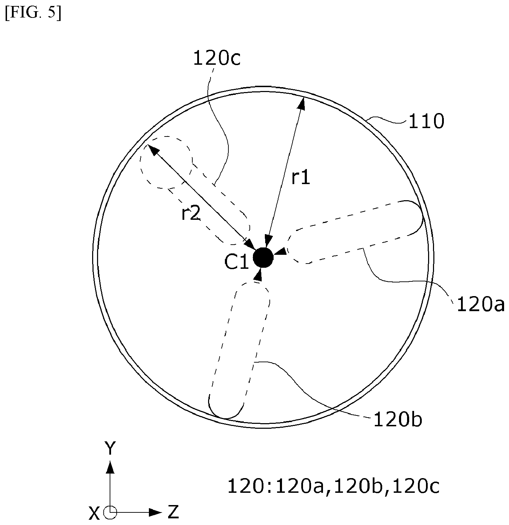

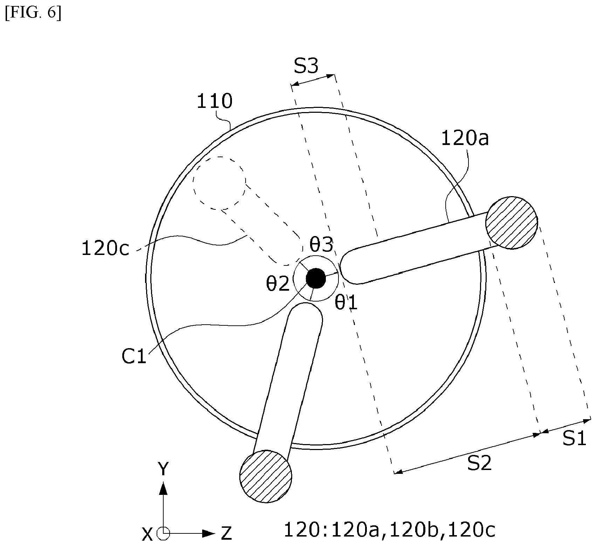

FIG. 4 is a diagram according to a first state of a catheter according to a first embodiment, FIG. 5 is a view cut along AA' in FIG. 4, FIG. 6 is a diagram of the catheter viewed from direction A1 in FIG. 4,

Referring to FIGS. 4 to 6, the first area S1 of the electrode support portion 120 may be partially exposed as in the catheter according to an embodiment, the first tube 110 moves in the proximal direction. For example, the electrode support portion exposed in the first area S1 of the electrode support portion 120 may be different depending on the length. As shown, the first electrode support portion 120a and second electrode support portion 120b may be exposed, and the third electrode support portion 120c may not be exposed. The second area S2 and third area S3 of the electrode support portion 120 may be located inside the first tube 110. That is, the second area S2 and third area S3 may not be exposed.

When the first tube 110 moves in the proximal direction and the first area S1 is exposed, the opening area of the electrode support portion 120 may be larger than the opening area of the first tube 110. For example, the radius r2 of the first area S1 of the exposed electrode support portions 120a and 120b may be larger than the radius r1 of the first tube 110 based on the center C1.

Furthermore, based on the center C1 of the first tube 110, each electrode support portions 120a to 120c or respective electrodes 130a to 130c may be located to have the same angles θ₁, θ₂, θ₃. That is, the first electrode support portion 120a to third electrode support portion 120c may be located at an angle of 120 degrees with respect to the center C1 of the first tube 110. Likewise, the first electrode 130a to third electrode 130c may be located at an angle of 120 degrees with respect to the center C1 of the first tube 110. The angle may change depending on the number of electrodes. Furthermore, the above description may be equally applied to various embodiments and various states described later.

Furthermore, in this specification, a direction toward the center C1 of the first tube 110 is described as the inside, and a direction opposite to the inside is described as the outside. For example, based on the center C1 of the first tube 110, the third area S3, second area S2, and first area S1 may be sequentially located outward (some overlapping areas may exist, but are excluded).

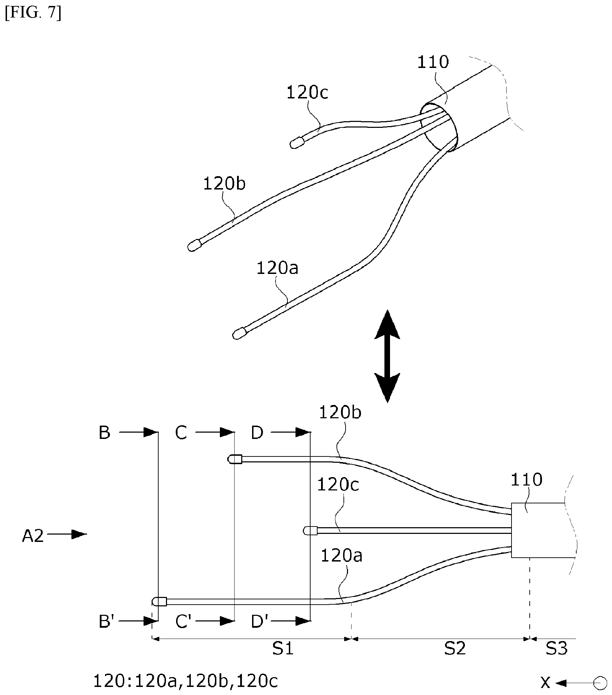

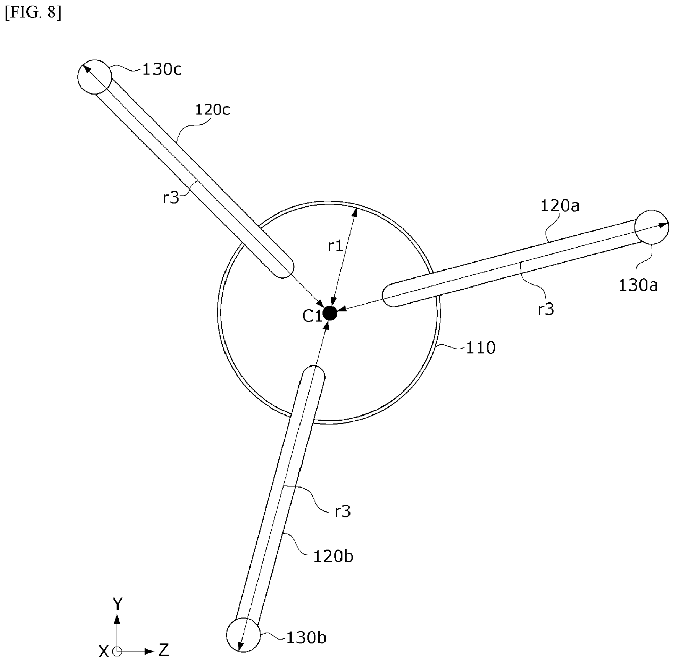

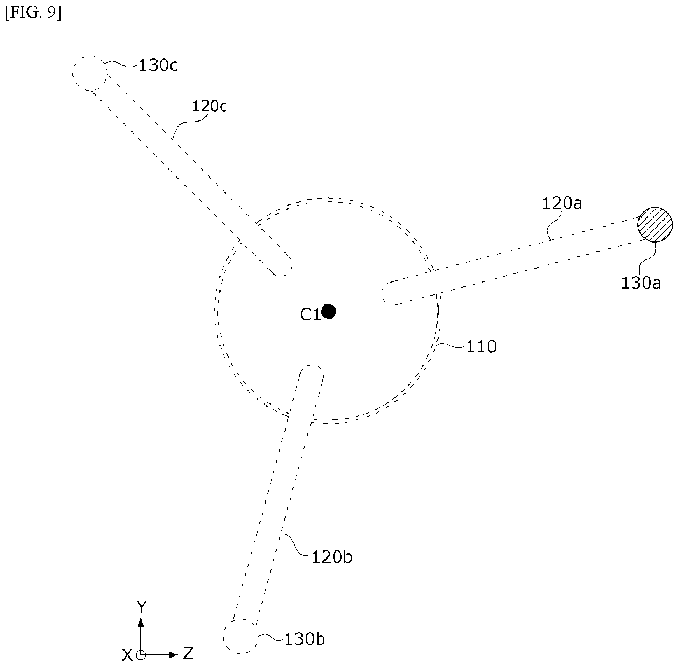

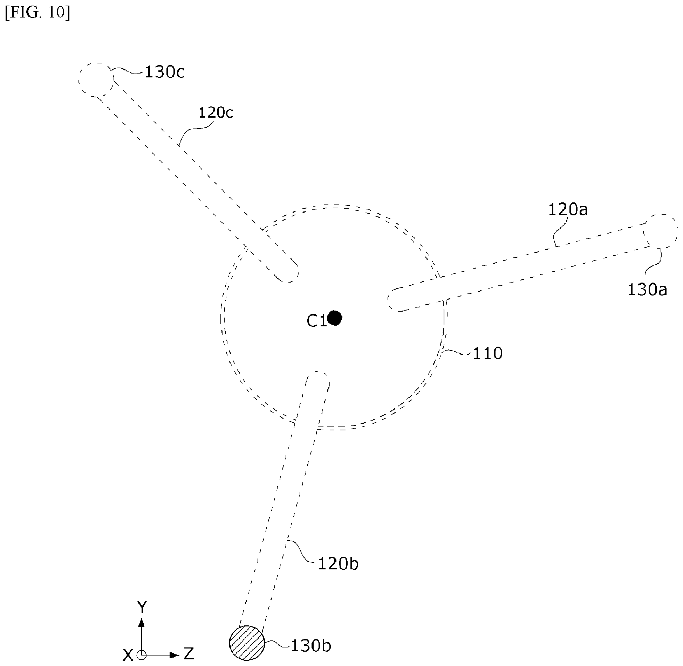

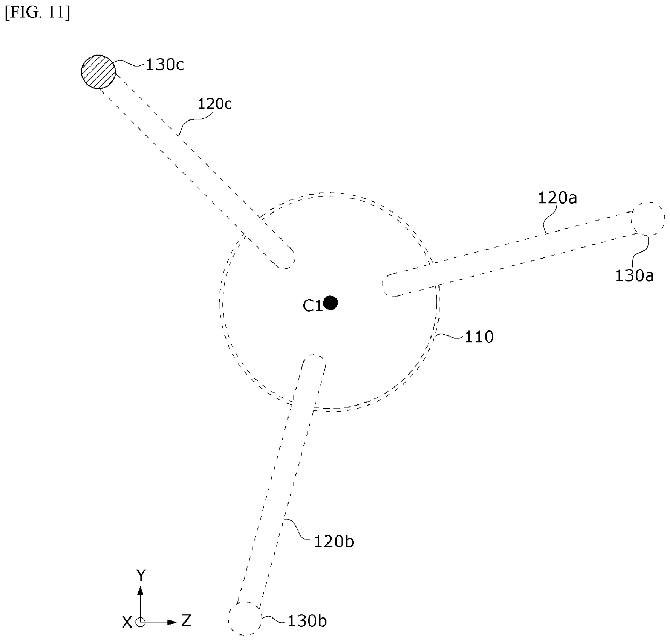

FIG. 7 is a diagram according to a second state of a catheter according to a first embodiment, FIG. 8 is a diagram of the catheter viewed from direction A2 in FIG. 7, FIG. 9 is a view cut along BB' in FIG. 7, FIG. 10 is a view cut along CC' in FIG. 7, FIG. 11 is a view cut along DD' in FIG. 7.

Referring to FIGS. 7 to 11, the second area S2 of the electrode support portion 120 may be partially exposed as in the catheter according to an embodiment, the first tube 110 moves in the proximal direction. In this case, the entire first area S1 of the electrode support portion 120 may be exposed. As shown, the first area S1 of the first to third electrode support portions 120a to 120c may be exposed.

In addition, as the first tube 110 moves in the proximal direction, the second area S2 of the first to third electrode support portions 120a to 120c may be exposed by the same length or area.

In addition, a portion of the second area S2 or third area S3 of the electrode support portion 120 may be located inside the first tube 110. That is, a portion of the second area S2 or third area S3 may not be exposed.

In an embodiment, when the first area S1 is fully exposed and the second area S2 begins to be exposed, as the first tube 110 moves in the proximal direction, the opening area of the electrode support portion 120 may be larger than the opening area of the first tube 110. That is, the opening area of the electrode support portion 120 may change in response to the extent of exposure of the second area S2. In other words, the opening area of the electrode support portion 120 may change in the second area S2. In the third area S3, the opening area of the electrode support portion 120 may remain unchanged even when the first tube 110 is moved (pulled proximally). In addition, in the first area S1, the opening area may or may not change due to movement of the first tube. In an embodiment, as exposure of the second area S2 increases, the opening area of the electrode support portion 120 may increase. Conversely, when the electrode support portion 120 is pulled proximally or the first tube 110 is moved in the distal direction while the second area S2 is exposed, the opening area of the electrode support portion 120 may be reduced. In addition, based on the center C1 of the first tube 110, the radius r1 of the first tube 110 may be smaller than the radius r3 for the electrode support portion 120 or first area S1 of the electrode support portion 120.

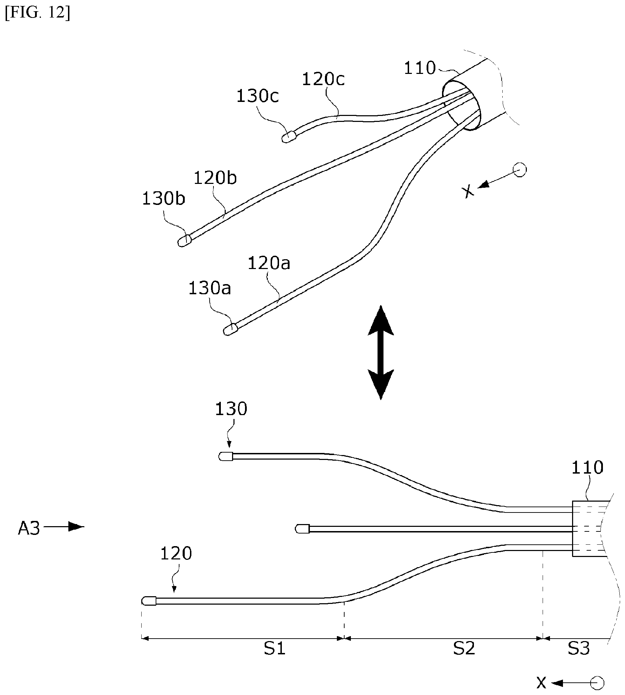

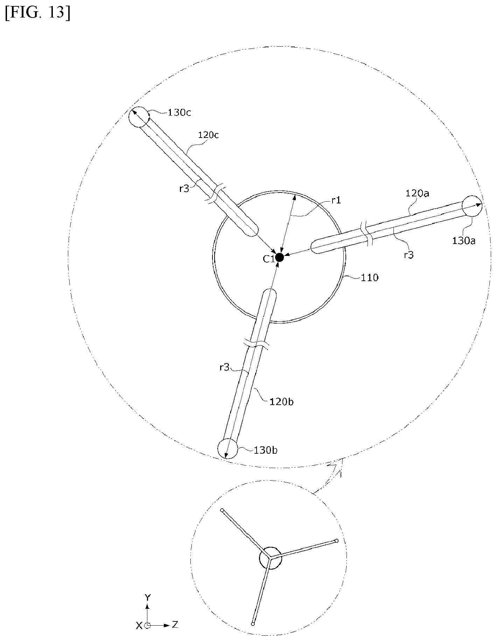

FIG. 12 is a diagram according to a third state of a catheter according to a first embodiment, FIG. 13 is a diagram of the catheter viewed from direction A3 in FIG. 12.

Referring to FIGS. 12 and 13, the third region S3 of the electrode support portion 120 may be partially exposed as in the catheter according to an embodiment, the first tube 110 moves in the proximal direction. In this case, both the first area S1 and second area S2 of the electrode support portion 120 may be exposed. As shown, the first area S1 and second area S2 of the first to third electrode supports 120a to 120c may be exposed. In addition, a portion of the third region S3 of the first to third electrode supports 120a to 120c may also be exposed.

In addition, as the first tube 110 moves in the proximal direction, the third region S3 of the first to third electrode supports 120a to 120c may be exposed by the same length or area.

Further, a portion of the third area S3 of the electrode support portion 120 may be located inside the first tube 110. That is, part of the third area S3 may not be exposed.

In an embodiment, when both the first area S1 and the second area S2 are exposed and the third area S3 begins to be exposed, even if the first tube 110 moves in the proximal direction, the opening area of the electrode support portion 120 may be maintained. In this state, the opening area of the electrode support portion 120 may be larger than the opening area of the first tube 110. The opening area of the electrode support portion 120 may be maintained regardless of the extent of exposure of the third region S3.

In addition, the opening area of the electrode support portion 120 changes only in the first area S1 or second area S2 and may be unrelated to the extent of exposure in the third area S3. Accordingly, as described above, in the third area S3, the opening area of the electrode support portion 120 may remain unchanged even when the first tube 110 moves.

In addition, based on the center C1 of the first tube 110, the radius r1 of the first tube 110 may be smaller than the radius r3 of the electrode support portion 120 or first area S1 of the electrode support portion 120.

Alternatively, in a structure in which the first area S1 extends inward, the opening area of the electrode support portion 120 changes in the first area S1 and second area S2, and may maintain regardless of the extent of exposure in the third area S3.

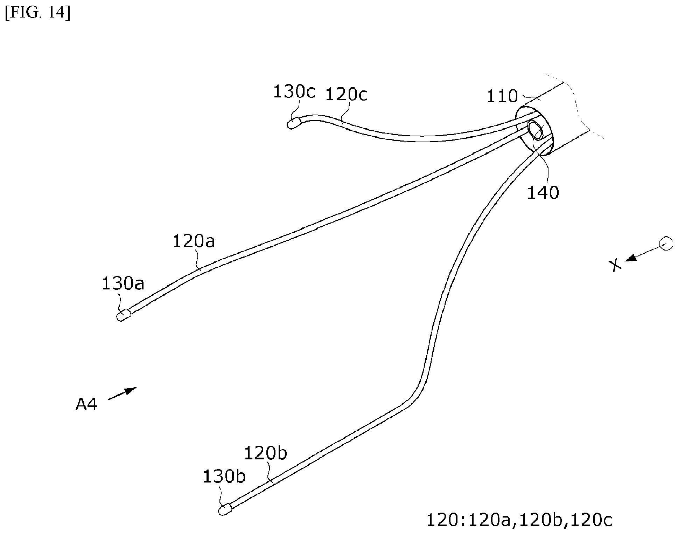

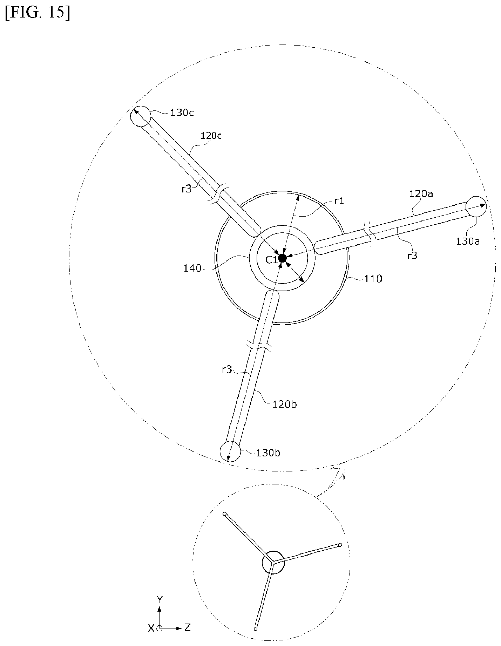

FIG. 14 is a diagram of a catheter according to a modified example, and FIG. 15 is a diagram of the catheter viewed from direction A4 in FIG. 14.

Referring to Figures 14 and 15, the catheter according to an embodiment may include the first tube 110, the electrode support portion 120, the electrode portion 130, and a second tube 140. Except for the description provided below, the above descriptions may be applied to the first tube 110, the electrode support portion 120, and the electrode portion 130.

The second tube 140 may be disposed inside the first tube 110. That is, the second tube 140 may be located within the hollow of the first tube 110. Further, the diameter of the second tube 140 may be smaller than the diameter of the first tube 110. Further, the center C1 of the first tube 110 may correspond to the center of the second tube 140.

The second tube 140 may be a guide tube. Accordingly, a guide wire, etc. may be inserted into the second tube 140. That is, the second tube 140 may be a component for passing a guide wire. Further, the catheter may be moved into the desired blood vessel through the guide wire.

In an embodiment, the electrode support portion 120 may be located between the first tube 110 and the second tube 140. Accordingly, the radius of the second tube 140 may be smaller than the radius of the electrode support portion based on the center C1 of the first tube 110.

In addition, the second tube 140 may be located at least partially in contact with the electrode support portion 120.

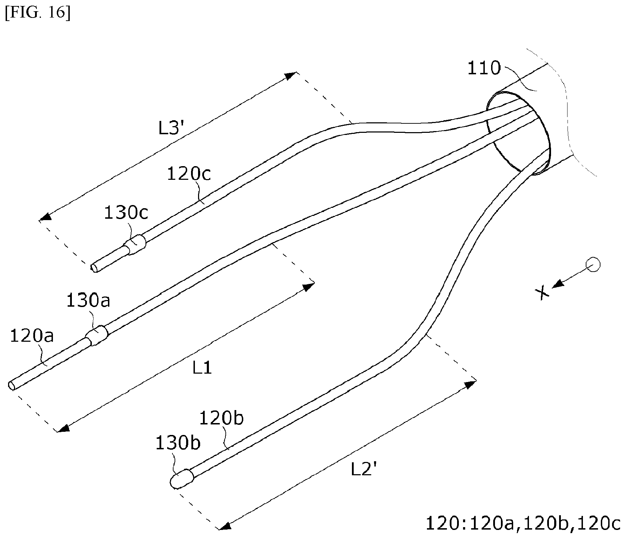

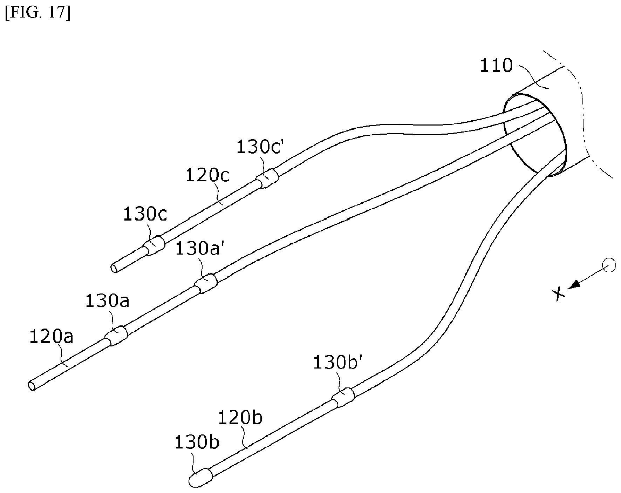

FIG. 16 is a diagram of a catheter according to a second embodiment, and FIG. 17 is a diagram of a catheter according to another modified example.

Referring to FIG. 16, the catheter according to a second embodiment may include the first tube 110, the electrode support portion 120, and the electrode portion 130. Except for the descriptions provided below, the above descriptions may be applied to the first tube 110, electrode support portion 120, and the electrode portion 130.

In this embodiment, the electrode support portion 120 may include the first electrode support portion 120a, the second electrode support portion 120b, and the third electrode support portion 120c, as described above.

In addition, the lengths of the plurality of electrode supports in the first direction (X-axis direction) of the first area S1 may all be the same. For example, the length L1 of the first area S 1 in the first electrode support portion 120a may be equal to the length L2' of the first area S1 in the second electrode support portion 120b and the length L3' of the first area S1 in the third electrode support portion 120c. Accordingly, the plurality of electrode support portions may be easily manufactured and mounted. Furthermore, the weight of the catheter is evenly distributed based on the center C1 of the first tube 110, so that procedures, etc. may be easily performed.

Referring to FIG. 17, in the catheter according to a modified example, except for the descriptions provided below, the above descriptions may be applied to the first tube 110, electrode support portion 120, and electrode portion 130.

As in the catheter according to the second embodiment, the lengths of the first areas S 1 of the electrode support may all be the same. Furthermore, there may be a plurality of electrodes disposed on each electrode support portion. For example, there may be a plurality of first electrodes 130a and 130a' mounted on the first electrode support portion 120a. In addition, there may be a plurality of second electrodes 130b and 130b' mounted on the second electrode support portion 120b. In addition, there may be a plurality of third electrodes 130c and 130c' mounted on the third electrode support portion 120c.

Furthermore, the plurality of electrodes mounted on each electrode support portion may be spaced apart in the longitudinal direction. In addition, the plurality of electrodes mounted on the electrode support portion 120 may be disposed to be offset from each other in the plane YZ intersecting the first direction (X-axis direction). For example, the first electrodes 130a and 130a', second electrodes 130b and 130b', and third electrodes 130c and 130c' may be disposed to be offset from each other in the plane YZ intersecting the first direction (X-axis direction). Alternatively, the first electrodes 130a and 130a', second electrodes 130b and 130b', and third electrodes 130c and 130c' may not overlap each other in the plane YZ intersecting the first direction (X-axis direction). With this configuration, the electrode support portion 120 where the electrode portion 130 is located may be easily located inside the first tube 110 or between the first tube 110 and the second tube (not shown). In case that the plurality of electrodes overlap on the plane YZ, the radius or diameter of the first tube 110 increases.

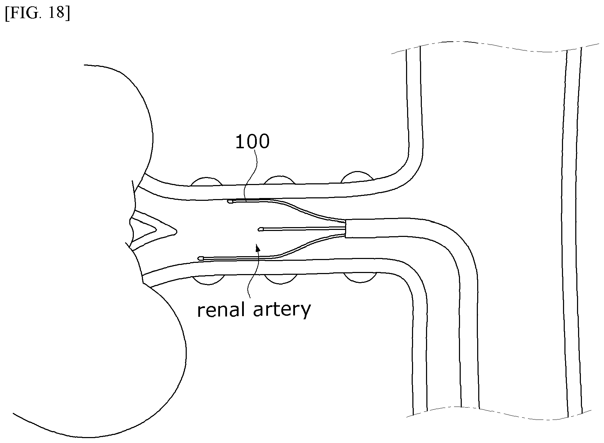

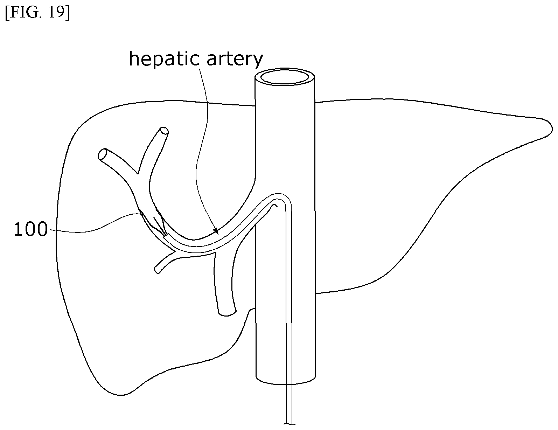

FIG. 18 is an illustrative example of the catheter inserted into the renal artery according to an embodiment, and FIG. 19 is an illustrative diagram of the catheter inserted into the hepatic artery according to an embodiment.

Referring to FIGS. 18 and 19, the catheter 100 according to an embodiment may provide the first tube 110 or second tube (not shown) with the plurality of electrodes and a small radius or diameter as described above. Accordingly, the catheter may be easily inserted into blood vessels with a small radius or diameter, and procedures such as resection may be performed within the blood vessel. Furthermore, since the first tube 110 and electrode support portion 120 are made of a flexible material, as shown in FIG. 19, the catheter according to an embodiment may easily move within blood vessels even if a blood vessel with a small radius or diameter has a curved or tortuous structure.

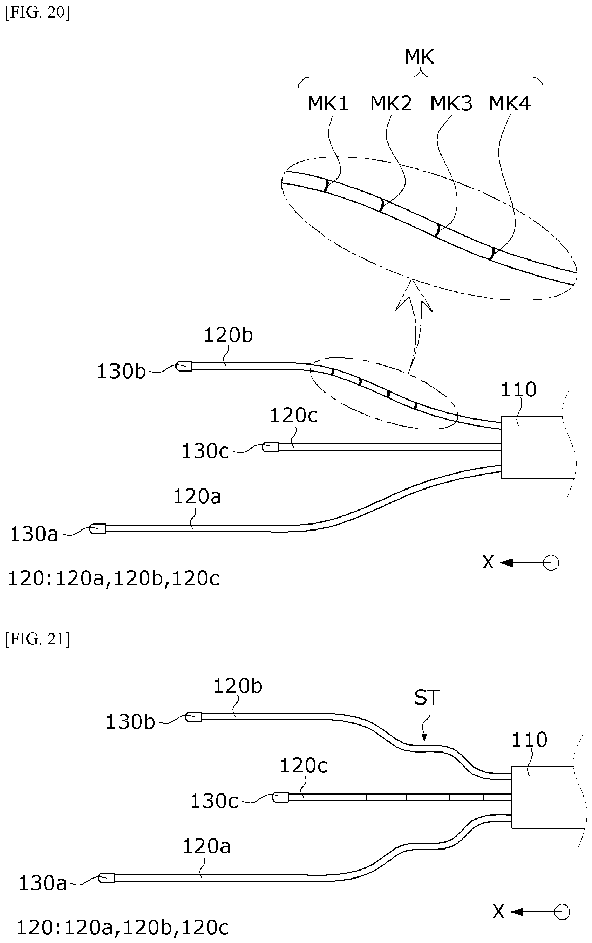

FIG. 20 is a diagram of a catheter according to a third embodiment.

Referring to FIG. 20, the catheter according to a third embodiment may include the first tube 110, the electrode support portion 120, and the electrode portion 130. Except for the description provided below, the above descriptions may be applied to the first tube 110, electrode support portion 120, and electrode portion 130.

In this example, a marker MK may be disposed in the second area of the electrode support portion. There may be a plurality of markers MKs. For example, the marker MK may include a first marker MK1, a second marker MK2, a third marker MK3, and a fourth marker MK4.

In addition, the plurality of markers MKs may be disposed at predetermined intervals in the second area S2. The predetermined interval may be increased, decreased or maintained proximally or distally. For example, a plurality of markers MKs may be disposed at equal intervals from each other.

In addition, as described above, the opening area of the electrode support portion may vary depending on the extent of exposure of the second area S2, or first area S1 and second area S2. Likewise, the electrode may be unfolded outward or folded inward depending on the extent of exposure of the second area S2.

For example, the first marker MK may be disposed closest to the first area S1. Further, the fourth marker MK4 may be disposed closest to the third area S3. In this case, the user may identify through the marker whether the first marker, second marker, etc. is exposed depending on the extent of exposure of the second area while the catheter is located within the blood vessel. Through this, the user may determine the opening area of the electrode support portion. In other words, the user may easily perceive the extent to which the first area of the electrode support portion is spread. Accordingly, the procedure may be performed while determining the extent to which the first area is spread so that nerve resection, etc. may be easily performed.

FIG. 21 is a diagram of a catheter according to a fourth embodiment.

Referring to FIG. 21, the catheter according to a fourth embodiment may include the first tube 110, the electrode support portion 120, and the electrode portion 130. Except for the descriptions provided below, the above descriptions may be applied to the first tube 110, electrode support portion 120, and electrode portion 130.

In this case, the second area S2 of the electrode support may have an inclined structure and a stepped structure. For example, the second area S2 of the electrode support portion may have a step portion ST. The step portion ST may have a structure in which an area extending inward and proximally from the first area S1 toward the third area S3 and an area extending proximally are repeated. With this configuration, the user may increase the opening area of the electrode support step by step even if the extent of exposure of the second area S2 increases. Accordingly, since the opening area is maintained at a predetermined moving distance even if the first tube 110 is moved proximally, the user may accurately perform nerve resection with a desired opening area.

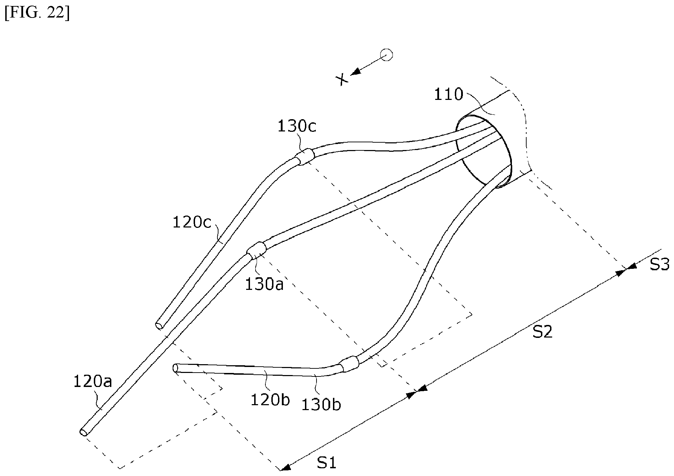

FIG. 22 is a diagram of a catheter according to a fifth embodiment.

Referring to FIG. 22, the catheter according to a fifth embodiment may include the first tube 110, the electrode support portion 120, and the electrode portion 130. Except for the descriptions provided below, the above descriptions may be applied to the first tube 110, electrode support portion 120, and electrode portion 130.

In this embodiment, the electrode support portion 120 may include the first electrode support portion 120a, the second electrode support portion 120b, and the third electrode support portion 120c, as described above.

In this case, the first area S1 in each electrode support portion 120 may extend inward. Accordingly, the respective electrode support portions 120a, 120b, and 120c extend toward the center of the first tube 110, so that the area, diameter, or width formed by each of the electrode support portions 120a, 120b, and 120c may decrease toward the distal end. With this configuration, problems such as the end of each electrode support portion piercing tissues, nerves, etc. located outside the electrode support portion during the procedure may be resolved. Thereby, patient safety during surgery may be further improved.

Furthermore, the lengths of the second areas S2 in the electrode support portion of the catheter according to the fifth embodiment may be different from each other. The lengths of the second areas of each of the first electrode support portion 120a, second electrode support portion 120b, and third electrode support portion 120c may be different. For example, each of the second areas S2 of the first electrode support portion 120a, second electrode support portion 120b, and third electrode support portion 120c may be small due to aberration. With this configuration, the first electrode 130a, second electrode 130b, and third electrode 130c may be disposed to be offset from each other in the plane YZ intersecting the first direction (X-axis direction). Alternatively, the first electrode 130a, second electrode 130b, and third electrode 130c may not overlap each other in the plane YZ intersecting the first direction (X-axis direction). With this configuration, the electrode support portion 120 where the electrode portion 130 is located may be easily located inside the first tube 110. In case that the plurality of electrodes overlap on the plane YZ, the radius or diameter of the first tube 110 may increase. Accordingly, the catheter 100 according to an embodiment may provide the first tube 110 with the plurality of electrodes and a small radius or diameter. Further, the catheter 100 may be easily inserted into the blood vessels with a small radius or diameter. Accordingly, procedures such as resection within blood vessels may be performed more easily.

In addition, the length of the plurality of electrode support portions in the first direction (X-axis direction) of the first area S1 may all be the same. For example, the length of the first area S1 in the first electrode support portion 120a may be equal to the length of the first area S1 in the second electrode support portion 120b and the length of the first area S1 in the third electrode support portion 120c. Accordingly, the plurality of electrode support portions may be easily manufactured and mounted.

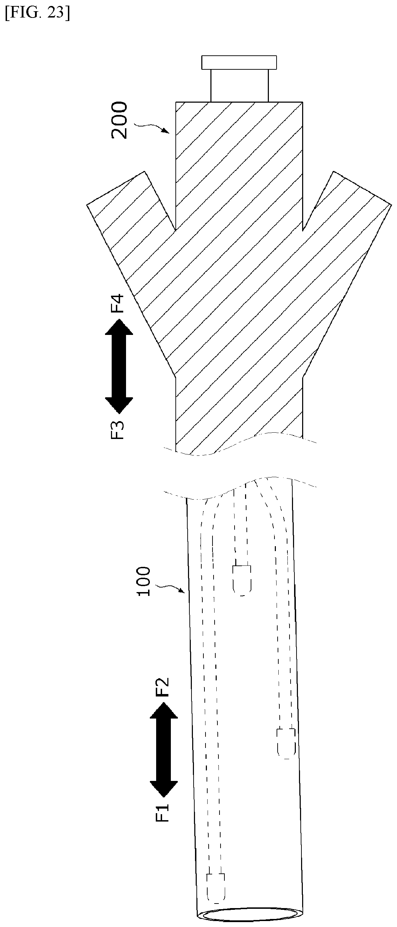

FIG. 23 is a diagram for explaining operations of a catheter and handle according to an embodiment.

Referring to FIG. 23, the electrode support portion may be expanded or non-extended by moving the first tube (F1, F2) or moving the handle 200 (F3, F4) as described above. The handle 200 is connected to the first tube or electrode support portion such that movement of the handle 200 may correspond to moving the first tube or electrode support portion proximally or distally, respectively.

In addition, the electrode support portion may be expanded, for example by moving the first tube proximally (F2). That is, the opening area of the electrode support portion may increase. In addition, the first tube may be moved to another treatment site only with the electrode support portion located within the first tube. That is, when finding the location to perform resection (procedure location), the electrode support portion may be inserted into the first tube by moving only the electrode support portion proximally (F2) while maintaining the first tube, or moving the first tube distally (F1) while fixing the electrode support portion at the handle side.

In addition, for example, by moving or pulling (F2) the first tube to the proximal end, the electrodes of the catheter 100 may be extended to the outside of the first tube or into the blood vessel. In this case, the handle 200 may be combined with the first tube or electrode support portion of the catheter 100. Accordingly, the user may spread the electrode support portion and the electrode outward by easily manipulating the handle 200 and catheter 100.

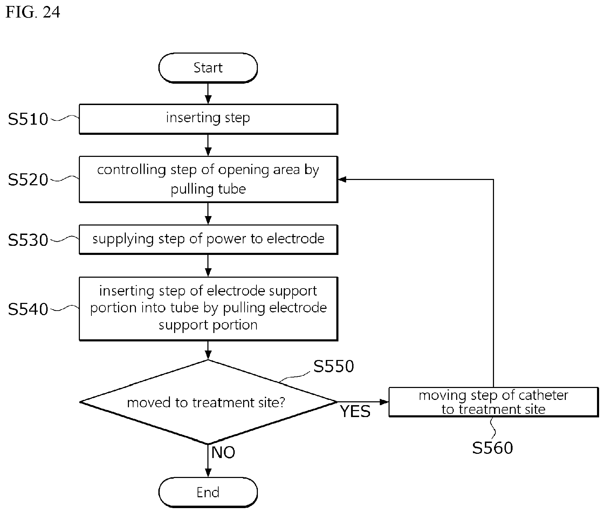

FIG. 24 is a flowchart of a method for operating a catheter according to an embodiment.

Referring to FIG. 24, a method for operating a catheter according to an embodiment may include inserting the first tube (S510), controlling the opening area of the electrode support portion in the first tube by pulling the first tube proximally (S520), and supplying power to the electrode (S530).

That is, the catheter (or first tube) may be inserted into a blood vessel, etc. by the user. Also, when the catheter is located in a desired blood vessel, the electrode support portion may be spread outward by pulling the first tube. As described above, the opening area of the electrode support portion may be controlled depending on the extent to which the first or second area of the electrode support portion is exposed. For example, considering the diameter of the blood vessel or the treatment site, the electrode support portion may be expanded by controlling the extent of exposure of the first or second area.

Next, power may be supplied to the electrode. Accordingly, energy can be transferred to the nerves around blood vessels through the electrode. Further, by the energy transferred, nerve around blood vessels may be ablated.

Furthermore, the method for operating a catheter according to an embodiment may further include the steps of inserting the first tube into the electrode support portion by pulling the electrode support portion (S540), determining whether to move to the treatment site (S550), and moving the catheter to the treatment site (S560).

That is, after the operator supplies power to the electrode at the treatment site (S530), the electrode support portion may be pulled proximally and inserted into the first tube. Accordingly, the catheter may be in the same state as at the insertion step. In this case, it may be determined whether to move the catheter to another treatment site (S550). Alternatively, the step of inserting the electrode support portion into the tube by pulling the electrode support portion (S540) may be replaced with a method of inserting the electrode support portion into the tube by moving the tube in the distal direction while the electrode support portion is fixed.

If there is no need to move to the treatment site, the catheter may be pulled out from the body.

In contrast, if movement to another treatment site is necessary, the catheter may be moved to the desired treatment site (S560). Then, the catheter may be operated by returning to the step of controlling the opening area by pulling the first tube described above (S520).

The term "-unit" used herein refers to a software element or a hardware element such as a field-programmable gate array (FPGA) and an application-specific integrated circuit (ASIC), and a "-unit" plays a certain role. However, a "-unit" is not limited to software or hardware elements. A "-unit" may be configured to be in an addressable recording medium or may also be configured to be reproduced by one or more processors. Accordingly, for example, a "-unit" include an element such as a software element, an object-oriented software element, a class element, and a task element, a process, a function, a feature, a procedure, a sub-routine, a segment of a program code, a driver, firmware, a microcode, a circuit, data, a database, a data structure, a table, an array, and a variable. Functions provided in elements and "units" may be combined into a smaller number of elements and "-units" or may be further divided into a larger number of elements and "-units". In addition, the elements and the "-units" may also be implemented to reproduce one or more central processing units (CPUs) within a device or a security multimedia card.

In the above description, the present invention has been described based on the exemplary embodiments, but the exemplary embodiments are for illustrative, and do not limit the present invention, and those skilled in the art will appreciate that various modifications and applications, which are not exemplified in the above description, may be made without departing from the scope of the essential characteristic of the present exemplary embodiments. For example, each constituent element specifically present in the exemplary embodiment may be modified and carried out. Further, the differences related to the modification and the application should be construed as being included in the scope of the present invention defined in the accompanying claims.

## Claims

1. A catheter, comprising:
a first tube; and
an electrode support portion disposed inside the first tube,
wherein an opening area of the electrode support portion changes according to an extent to which a predetermined area of the electrode support portion is exposed by pulling the first tube proximally.

2. The catheter of claim 1, wherein the catheter comprises an electrode portion disposed on the electrode support portion.

3. The catheter of claim 2, wherein the electrode support portion has an opening area that changes after the electrode portion is exposed.

4. The catheter of claim 2, wherein the electrode support portion includes:
a distally disposed first area;
a second area in contact with a proximal end of the first area and extending proximally and inside of the first tube;
a third area in contact with a proximal end of the second area and extending proximally.

5. The catheter of claim 4, wherein the electrode support portion includes a plurality of electrode support portions,
wherein the plurality of electrode support portions includes a first electrode support portion, a second electrode support portion, and a third electrode support portion spaced apart from each other.

6. The catheter of claim of claim 5, wherein the first area of the first electrode support portion has a length equal to or different from at least one of the first area of the second electrode support portion and the first area of the third electrode support portion in a longitudinal direction.

7. The catheter of claim 5, wherein the electrode portion includes a plurality of electrode portions,
wherein the plurality of electrodes includes:
a first electrode disposed in the first area of the first electrode support portion;
a second electrode disposed in the first area of the second electrode support portion; and
a third electrode disposed in the first area of the third electrode support portion.

8. The catheter of claim 7, wherein the first electrode, the second electrode, and the third electrode are disposed to be spaced apart in a longitudinal direction.

9. The catheter of claim 7, wherein the first electrode, the second electrode, and the third electrode are disposed to be offset from each other in a plane intersecting a longitudinal direction.

10. The catheter of claim 5, wherein the first electrode support portion, the second electrode support portion, and the third electrode support portion are disposed to have the same angle with respect to a center of the first tube.

11. The catheter of claim 1, wherein the catheter comprises a second tube disposed inside the first tube.

12. The catheter of claim 11, wherein the electrode support portion is disposed between the first tube and the second tube.

13. The catheter of claim 4, wherein the catheter further comprises a marker disposed in the second area.

14. The catheter of claim 13, wherein the marker is disposed along predetermined intervals.

15. The catheter of claim 4, wherein the opening area changes in the first area or the second area.
